# EUROPÄISCHE PATENTANMELDUNG

(11) **EP 1 273 309 A1**
(43) Veröffentlichungstag der Anmeldung: **08.01.2003**
(21) Anmeldenummer: 02010650.6
(22) Anmeldetag: 13.05.2002
(51) Int. Cl.: A61K 48/00, C12N 15/52, C12N 15/67, C12N 15/86, C12N 15/88, C12N 15/85, A61P 35/00

(54) **Gentherapeutisches Verfahren zur Behandlung von GnRH-Rezeptor-positiven Karzinomen durch GnRH induzierte tumorzellspezifische Aktivierung eines therapeutischen Gens, zugehörige Nukleinsäurekonstrukte und Vektoren**

(30) Priorität: 05.07.2001 DE 10132607
(71) Anmelder: Georg-August Universität Göttingen, 37075 Göttingen (DE)
(72) Erfinder: Emons, Günter, Prof. Dr. Med., 37085 Göttingen (DE); Gründker, Carsten, Dr. rer. nat., 37120 Bovenden (DE); Huschmand Nia, Abdolhamid, Dr. med., 37073 Göttingen (DE)
(74) Vertreter: Läufer, Martina, Dr.

(57) **Zusammenfassung**

Die GnRH-induzierte NF_{K}B Aktivierung kann dazu genutzt werden, ein eingebrachtes Effektorgen unter Kontrolle eines NF_{K}B-abhängigen Promotors tumorzellspezifisch zu exprimieren. Dazu werden die Tumorzellen mit einem Vektor, der das Effektorgen unter Kontrolle des NF_{K}B-Promotors enthält, transfiziert. Anschließend wird dieses Gen zielzellspezifisch durch die Aktivierung von NF_{K}B durch GnRH Agonisten aktiviert, so dass nur in GnRH-Rezeptor tragenden Tumorzellen ein inaktiver Pro-Wirkstoff (Pro-Drug) zu einem cytotoxischen Wirkstoff umgesetzt und somit eine zielzellspezifische cytotoxische Therapie ermöglicht wird.

## Beschreibung

Die Erfindung bezieht sich auf Nukleinsäurekonstrukte und diese enthaltende Vektoren zur Einschleusung in Zellen Gonadotropin-Releasing-Hormon (GnRH)-positiver Karzinome, und auf deren Verwendung zur Behandlung GnRH-positiver Tumore.

Das Endometriumkarzinom ist die häufigste gynäkologische Krebserkrankung in der westlichen Welt. In den meisten Fällen wird ein Endometriumkarzinom in einem frühen Stadium diagnostiziert, so daß ein operativer Eingriff oder eine Chemotherapie recht gute Heilungschancen bringen. Steroid-Rezeptor negative Tumore bei älteren Frauen oder spätere Krebsstadien haben sehr ungünstige Prognosen. Bisher existiert keine Therapie um diese ungünstigen Prognosen zu verbessern.

Ovarialtumore treten seltener auf, haben aber sehr viel ungünstigere Prognosen und führen häufig zum Tod. Das Ovarialkarzinom tritt zwar relativ selten auf, verursacht aber mehr Todesfälle als alle übrigen gynäkologischen Karzinome zusammengenommen. Effektive Operationstechniken und Chemotherapieverfahren sind zwar etabliert, aber für fortgeschrittene Stadien oder für Rückfälle gibt es kaum Heilungschancen.

Das Mammakarzinom ist eine der häufigsten malignen Erkrankungen der Frau. Etwa 10% aller Frauen erkranken während ihres Lebens an Brustkrebs, was ungefähr 25% aller Krebstodesfälle der Frau entspricht. In den letzten 10 Jahren hat die gesamte Behandlung, sowohl die operative als auch die medikamentöse Therapie eine wesentliche Veränderung erfahren. Dies führte trotz leicht ansteigender Tendenz der Brustkrebsrate, zwar zu einem Rückgang der Gesamtsterblichkeit dieser Erkrankung, aber für fortgeschrittene Stadien oder für Rückfälle gibt es auch beim Mammakarzinom wenig Heilungschancen.

Sowohl für das Endometriumkarzinom, das Ovarialkarzinom als auch für das Mammakarzinom werden neue, gut verträgliche und vor allem effizientere Therapien benötigt.

Die Aufgabe der Erfindung besteht darin, Mittel für die Behandlung der o.a. Tumorarten zur Verfügung zu stellen.

Diese Aufgabe wird gelöst durch die Bereitstellung eines Nukleinsäurekonstruktes, welches ein therapeutisches Gen, dessen Genprodukt in einer Zelle die Erzeugung eines zytotoxischen Wirkstoffs auslöst, unter Kontrolle eines Nukleus-Faktor-kappa-B- (NFκB)-spezifischen Promotors enthält. Bei der Tumorbehandlung wird dieses Konstrukt mit gentherapeutischen Mitteln in Tumorzielzellen eingeschleust, wobei das genaue Verfahren nachfolgend noch näher beschrieben wird.

Über 80% der Endometrium- und Ovarialkarzinome sowie über 50% der Mammakarzinome exprimieren das Gonadotropin Releasing Hormon (GnRH) und seinen Rezeptor als Teil eines autokrinen Regulationsmechanismus der Zellproliferation, der Wachstums- und Differenzierungskontrolle. Dagegen exprimieren nur wenige reproduktive Organe sowie Hypothalamus und Hypophyse den GnRH Rezeptor. Daher eignet sich der GnRH Rezeptor besonders gut als Target für eine tumorzellspezifische Therapie.
Die Signaltransduktion des GnRH Rezeptors in gynäkologischen Tumoren unterscheidet sich grundsätzlich von derjenigen in Hypothalamus und Hypophyse (Gründker et al. 2002). GnRH und seine Analoga induzieren nur in Endometrium- und Ovarialkarzinomzellen GnRH Rezeptor vermittelt einen 5- bis 8-fachen Anstieg der Aktivität des Transkriptionsfaktors Nukleus Faktor κB (NFκB).

Die GnRH-induzierte NFκB Aktivierung wird nun erfindungsgemäß dazu genutzt, ein eingebrachtes Effektorgen (im weiteren auch als therapeutisches Gen bezeichnet) unter Kontrolle eines NFκB-abhängigen Promotors tumorzellspezifisch zu exprimieren. Dazu werden die Tumorzellen mit einem Nukleinsäurekonstrukt, das das Effektor-Gen unter Kontrolle des NFκB-Promotors vorzugsweise in einem Vektor enthält, transfiziert. Anschließend kann dieses Gen zielzellspezifisch durch die Aktivierung von NFκB durch GnRH-Analoga aktiviert werden, so daß nur in GnRH-Rezeptor tragenden Tumorzellen ein inaktiver Wirkstoff-Vorläufer (Pro-Drug) zu einem zytotoxischen Wirkstoff umgesetzt und somit eine zielzellspezifische zytotoxische Therapie ermöglicht wird.

Der proof of principle für diesen Ansatz konnte erbracht werden. In *in vivo* Versuchen mit tumortragenden Nacktmäusen, die liposomal mit dem Reportergen LacZ unter Kontrolle des NFκB-Promotors intraperitoneal transfiziert wurden, konnte gezeigt werden, daß NFκB durch das GnRH Analogon Triptorelin nur in GnRH Rezeptor positiven Tumorzellen aktiviert wurde. In Ovar und Uterus war NFκB konstitutiv aktiv. Ovar und Uterus werden aber bei entsprechenden Karzinomen vorher entfernt. Alle anderen Organe zeigten keine Aktivierung.

Diese Therapieform kann bei allen GnRH Rezeptor exprimierenden Karzinomen angewendet werden. Dazu gehören neben den schon erwähnten Endometrium-, Ovarial- (> 80% der Tumore exprimieren den GnRH Rezeptor) und Mammakarzinomen (> 50% der Tumore exprimieren den GnRH Rezeptor) auch Prostatakarzinome. Für letztere sind noch keine genaueren Zahlen bekannt, wieviel Prozent dieser Tumore den GnRH-Rezeptor exprimieren.

Grundsätzlich muss das therapeutische Gen innerhalb des erfindungsgmäßen Konstrukts in der Lage sein, in der Zielzelle die Erzeugung einer zytotoxischen Substanz, d.h. eines zytotoxischen Wirkstoffs auf irgendeine Weise auszulösen. Im allgemeinen wird dies dadurch bewirkt, dass das Genprodukt des therapeutischen Gens ein Enzym ist, welches einen in der Zelle vorhandenen bzw. dieser zugeführten Wirkstoff-Vorläufer in den zytotoxischen Wirkstoff umwandeln kann.

Das therapeutische Gen wird nur in GnRH-Rezeptor-positiven Zellen aktiviert, indem bei Ausschüttung oder Zuführung von GnRH oder eines GnRH-Analogons ein NFκBspezifischer Promotor die Aktivierung des Gens induziert. Der NFκB-spezifische Promotor enthält vorzugsweise wenigstens eine Kopie des kappaB-Enhancers, insbesondere 4 bis 6 Kopien, fusioniert an einen für die Aktivierung des therapeutischen Gens geeigneten Promotor.

Das der Erfindung zugrunde liegende Prinzip besteht darin, dass ein therapuetisches Gen oder Effektorgen unter Kontrolle des NFκB-Promotors steht, der tumorzellspezifisch über GnRH (LHRH) oder GnRH-Analoga in GnRH-Rezeptor-positiven Tumorzellen (Ovar-, Endometrium-, Mamma- und Prostatatumoren) aktiviert wird. Auf das einzelne therapeutische Gen kommt es nicht an, so dass für die Ausführung der Erfindung verschiedene Promotor-GenNorläufer-Wirkstoff-Paare geeignet sind.

Für die Krebstherapie handelt es sich bei den therapeutischen Genen in erster Linie um Suizidgene, die das Absterben der Zielzelle auslösen, wobei therapeutische Gene, die eine echte Therapie der Zielzelle (Rückprogrammierung der Krebszelle) ermöglichen, nicht ausgeschlossen werden.

Eine zusammenfassende Beschreibung zur Zeit gängiger Suizidgene findet sich beispielsweise in: G. Coukos, "Gene Therapy for Ovarian Cancer", Oncology 9, 2001, 1197 - 1207.

Beispiele für Suizidgene, die als therapeutische Gene im Rahmen dieser Erfindung zum Einsatz kommen können, sind in Tabelle 1 angegeben.

In einem besonders bevorzugten Ausführungsbeispiel ist vorgesehen, dass das therapeutische Gen das *Herpes simplex Virus* (HSV)-Thymidinkinase-Gen ist. Das HSV-TK-Gen wird unter die Kontrolle des HSV-TK-Promotors gestellt. HSV-TK konvertiert nicht-toxisches Ganciclovir und dessen Abkömmlinge in toxisches Ganciclovir-Triphosphat (bzw. dessen Abkömmlinge) (Vorteil: Bystander Effekt).

In einem weiteren Ausführungsbeispiel ist vorgesehen, dass das therapeutische Gen das *Varicella zoster* Virus-Thymidinkinase-Gen ist. VZV-TK konvertiert nicht-toxisches 6-Methoxypurin-Arabinonucleosid (araM) in toxisches Adenin-Arabinonucleosid-Triphosphat (araATP).

In einem anderen Ausführungsbeispiel ist vorgesehen, dass das therapeutische Gen das *Echerichia coli*-Cytosindeaminase-Gen ist. Bei Aktivierung durch einen geeigneten Promotor konvertiert E.coli-CD nicht-toxisches 5-Fluorocytosin in toxisches 5-Fluorouracil (Vorteil: Bystander-Effekt).

In der Regel liegt das Nukleinsäurekonstrukt in einem Plasmid vor. Die Minimalausstattung des Plasmids besteht aus mindestens einer Kopie, vorzugsweise 4 bis 6 Kopien, des κB-Enhancers, der eine NFκB-Bindungsstelle darstellt, fusioniert an einen Promotor, der spezifisch das therapeutische Gen induziert. Durch Fusionieren des KappaB-Enhancers mit dem Promotor erhält man einen NFκB-spezifischen Promotor für das therapeutische Gen. Das Konstrukt enthält weiterhin wenigstens eine PolyA-Sequenz.

In Weiterbildung der Erfindung kann das Konstrukt zusätzlich eine Sequenz für die Expression des Wirkstoff-Vorläufers in den Zielzellen enthalten. Diese umfasst i.a. wenigstens eine kodierende Sequenz für den Wirkstoff-Vorläufer und einen geeigneten Promotor. Ferner ist es auch möglich, für die Zufuhr des Wirkstoff-Vorläufers ein gesondertes Plasmid bzw. Nukleinsäurekonstrukt vorzusehen, welches wenigstens eine für den Wirkstoff-Vorläufer kodierende Sequenz und einen Promotor enthält.

Es besteht die Möglichkeit das Plasmid (Konstrukt) mittels verschiedener nicht-viraler (zB. liposomaler) Techniken in die Zielzellen einzuschleusen. Virale Transfektionsmöglichkeiten bestehen ebenfalls, sind aber mit einer Vielzahl von Nebenwirkungen behaftet. Die durch virale Techniken erreichbare gewisse Tumorspezifität (zB. Transfektion von proliferierenden Zellen) ist bei dem vorgestellten Therapieverfahren nicht notwendig, da über die spezifische Expression des GnRH (LHRH) Rezeptors und zusätzlich über den nur für GnRH-Rezeptor tragende Tumorzellen spezifischen Wirkungsmechanismus [Aktivierung von NFκB durch GnRH (LHRH) und seine Analoga] eine sehr viel höhere Tumorspezifität der Therapie gewährleistet ist. Grundsätzlich ist die Verwendung viraler Vektoren jedoch ebenfalls möglich.

In bevorzugter Ausführungsform wird das Konstrukt in einem liposomalen Vektor in die Tumor-Zielzellen eingeschleust. Kim et al. (Gynecologic Oncology 2002 Band 84 Seiten 85 bis 93) beschreiben beispielsweise ein effizientes auch hier verwendbares liposomales Verfahren, um humane Ovarialkarzinomzellen zu transfizieren.

Für ein Präparat zur Behandlung von GnRH-Rezeptor-positiven Tumoren kann das Nukleinsäurekonstrukt oder der Vektor gemäß der Erfindung beispielsweise in einer Injektionslösung oder einer Infusionslösung enthalten sein. Dieses Präparat kann zusätzlich den erforderlichen Wirkstoff-Vorläufer enthalten oder für die Co-Applikation mit diesem vorgesehen sein, sofern der Wirkstoff-Vorläufer nicht in der Zelle vorhanden oder auf dem Nukleinsäurekonstrukt kodiert ist.

Das Präparat mit dem Nukleinsäurekonstrukt oder dem Vektor der dieses Konstrukt enthält wird in zeitlicher Abstimmung mit GnRH oder einem GnRH-Analogon und gegebenenfalls dem Wirkstoff-Vorläufer oder einem für den Wirkstoff-Vorläufer kodierenden Plasmid verabreicht.

Die Tumorbehandlung erfolgt in einem bevorzugten Ausführungsbeispiel der Erfindung nach folgendem Schema:
a) Einschleusen des Nukleinsäurekonstrukts oder des Vektors nach einem der Ansprüche 1 bis 9 in Tumor-Zielzellen;
b) Verabreichung von GnRH oder einem GnRH-Analogon in zeitlichem Abstand zu Schritt a);.
c) Verabreichung eines Wirkstoff-Vorläufers, der durch das Genprodukt des in dem Nukleinsäurekonstrukt enthaltenen therapeutischen Gens in den Wirkstoff umgewandelt wird, in zeitlichen Abstimmung vor, nach oder mit Schritt b), sofern der Wirkstoff-Vorläufer nicht als natürliche zelluläre Substanz in der Zelle bereits vorhanden ist.

Das GnRH oder das GnRH-Analogon wird in einer vorteilhaften Verfahrensweise 8 bis 48 Stunden nach dem Einschleusen des Konstruktes oder des Vektors in die Zielzellen gegeben. Als GnRH-Analogon wird sehr vorteilhaft Triptorelin gegeben, vorzugsweise systemisch.

Das Einschleusen des nackten Konstruktes oder des Vektors kann durch intraperitoneale Injektion erfolgen. Die behandelbaren Tumore werden so gut erreicht. Die Verabreichung von GnRH oder einem GnRH-Analogon erfolgt anschließend vorzugsweise intraperitoneal oder intravenös. Der Wirkstoff-Vorläufer wird - sofern erforderlich - nach oder mit dem GnRH oder GnRH-Analogon vorzugsweise intraperitoneal verabreicht. Eine weitere Möglichkeit besteht darin, daß der Wirkstoff-Vorläufer zusätzlich auf dem Nukleinsäurekonstrukt kodiert ist.

In einem besonders bevorzugten Verfahrensbeispiel wird das Plasmid "pNFκB-TK verwendet. Das Plasmid " pNFκB-TK " wurde konstruiert, um durch NFκB, welches über GnRH (LHRH) und seine Analoga in GnRH (LHRH) Rezeptor positiven Tumorzellen spezifisch aktiviert wird, das Effektorgen Herpes simplex Virus Thymidinkinase (HSV-TK) zu exprimieren. Das Plasmid "pNFκB-TK" enthält 4 Tandemkopien des κB Enhancers fusioniert an den Herpes simplex Virus Thymidinkinase Promotor gefolgt vom Herpes simplex Virus Thymidinkinase Gen mit PolyA-Schwanz.

Das Plasmid "pNFκB-TK" wird zusammen mit einem Transfektionsreagenz auf Liposomenbasis intraperitoneal injiziert. Nach 24 Stunden wird ein GnRH (LHRH) Analogon injiziert. Die Bindung des GnRH (LHRH) Analogons an den GnRH (LHRH) Rezeptor der Tumorzelle führt zur Aktivierung von NFκB. Aktiviertes NFκB bindet and den κB Enhancer des Plasmids "pNFκB-TK" und induziert damit die Expression des Herpes simples Virus Thymidinkinase Gens. Das Genprodukt, die Herpes simplex Virus Thymidinkinase phosphoryliert nun den verabreichten Vorläuferwirkstoff Ganciclovir, der durch humane Enzyme nicht umgesetzt werden kann. Dadurch entsteht schließlich der zytotoxische Wirkstoff Ganciclovir-Triphosphat, der die Tumorzellen abtötet.

An normalen Zellen, die keine GnRH (LHRH) Rezeptoren exprimieren, kann GnRH (LHRH) und seine Analoga den NFκB nicht aktivieren. Außerdem ist der Mechanismus der NFκB Aktivierung durch GnRH (LHRH) und seine Analoga auf Tumore reproduktiver Organe beschränkt.

Im folgenden wird die Erfindung anhand von Beispielen näher beschrieben und es wird auf Figuren Bezug genommen. Im Einzelnen zeigen:

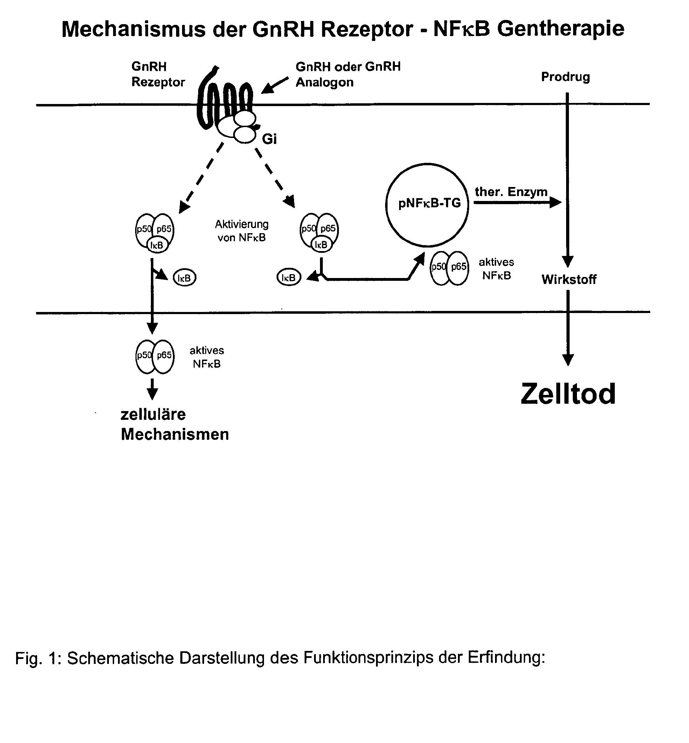

- Fig. 1: schematische Darstellung des Funktionsprinzips der Erfindung GnRH (LHRH) oder GnRH Analoga binden an den GnRH Rezeptor an der Zelloberfläche der Tumorzelle. Dadurch wird tumorspezifisch Nukleus Faktor kappa B (NFκB) aktiviert. Aktiviertes NFκB bindet an die κB-Bindungstelle des Plasmids und induziert dadurch die Expression des Effektorgens (z.B. Herpes simplex Virus Thymidinkinase Gen). Das Genprodukt ist ein Enzym (z.B. Herpes simplex Virus Thymidinkinase), welches die Umwandlung des Wirkstoffvorläufers (z.B. Ganciclovir) in einen Wirkstoff (z.B. Ganciclovir-Triphosphat) katalysiert. Dieser führt zum Tod der Tumorzelle.

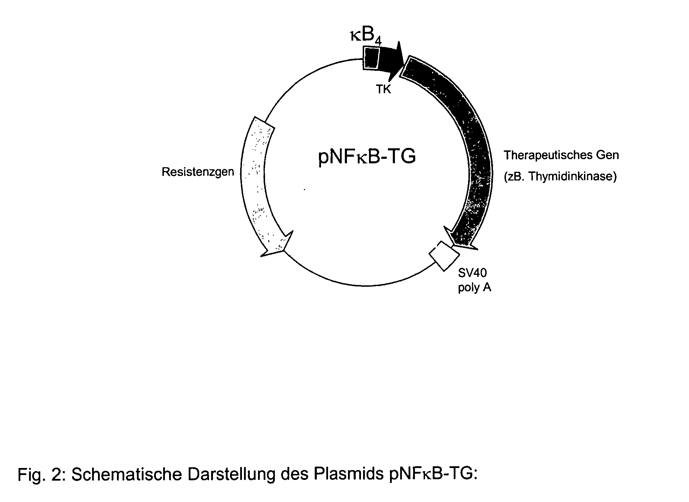

- Fig. 2: schematische Darstellung des Plasmids pNFκB-TG
- TG = therapeutisches Gen = Effektorgen (hier als Beispiel Herpes simples Virus Thymidinkinase)
- κB₄- TK= 4 Kopien des kappa B Enhancers fusioniert an einen für die Aktivierung des therapeutischen Gens geeigneten Promotors (hier TK = Thymidinkinase Promotor).
- SV40 poly A = Poly A Sequenz
- Resistenzgen

### EXPERIMENTALTEIL

### Nachweis von GnRH-Rezeptoren in Zellinien und Tumorgewebe

Um die Frequenz der Expression des GnRH Rezeptors in Endometrium- und Ovarialkarzinomen zu ermitteln, wurden verschiedene Endometrium- und Ovarialkarzinomzellinien sowie 40 Endometrium- und Ovarial-Primärkarzinome mittels eines spezifischen Radiorezeptorassays sowie mittels RT-PCR auf die Expression von GnRH-Bindungsstellen untersucht. Bei den meisten Ovarial- (EFO-21, EFO-27, NIH: Ovcar-3, BG-1) und Endometriumkarzinomzellinien (Ishikawa, HEC-1A, HEC-1B, KLE, AN-3-CA) konnte jeweils eine hochaffine Bindungsstelle für das GnRH Analogon Triptorelin (Kd 1,5 - 8,2 nmol/L) nachgewiesen werden. Die beiden Ovarialkarzinomzellinien SK-OV-3 und SW 626 und die Endometriumkarzinomzellinie MFE 296 reagierten negativ. Bei der Untersuchung der Primärtumore zeigten mehr als 80 % der Ovarial- und der Endometriumkarzinome hochaffine Bindungsstellen (Kd 0,1 - 90 nmol/L) für GNRH. Aufgrund dieser doch hohen Frequenz der GNRH Rezeptor Expression eignet sich der GnRH Rezeptor gut als Target für eine zielzellspezifische Therapie.

### Die meisten Normalgewebe exprimieren keine GnRH-Rezeptoren:

Voraussetzung für eine zielzellspezifische Tumortherapie via GnRH Rezeptor ist, daß Normalgewebe gar keine oder nur in einer geringen Frequenz GnRH Rezeptoren exprimieren. Durch RT-PCR und Radioliganden Assays konnte gezeigt werden, daß nur sehr wenige Normalgewebe den GnRH-Rezeptor exprimieren. GnRH-Rezeptor Expression wurde außer in Hypophyse und Hypothalamus ausschließlich in den reproduktiven Organen Ovar, Myometrium, Endometrium, Tube und Zervix sowie in der Plazenta und der Mamma gefunden (Kakar et al. 1994 und eigene unpublizierte Daten). Alle nicht-reproduktiven Organe einschließlich des lymphatischen und des blutbildenden Systems waren GnRH-Rezeptor negativ. Hypothalamus und Hypophyse werden durch eine lokale Therapie via GNRH Rezeptor (intrperitoneale Applikation) nicht tangiert. Desweiteren wird NFκB in hypophysären GnRH Rezeptor positiven Zellen nicht aktiviert (siehe unter den nächsten beiden Abschnitten). Außerdem würde aktiviertes NFκB in diesen nicht proliferierenden Zellen nicht toxisch sein. Die reproduktiven Organe wie Uterus und Ovar werden bei entsprechenden Karzinomen entfernt. Das Brustgewebe wird genauso wie Hypophyse und Hypothalamus nicht tangiert. Dadurch daß die überwiegende Mehrheit der Endometrium- und Ovarialkarzinome GnRH Rezeptoren exprimieren, aber nur sehr wenige Normalgewebe GnRH Rezeptoren besitzen scheint ein Targeting via GnRH-Rezeptor besonders für eine zielzellspezifische Therapie geeignet zu sein.

### Signaltransduktion des GnRH-Rezeptors im Tumor:

Die folgenden Experimente wurden durchgeführt, um die Unterschiede zwischen der Signaltransduktion in Hypophyse und Hypothalamus sowie in gynäkologischen Tumoren aufzuzeigen. Es wurden verschiedene Schlüsselstellen der Signaltransduktion des GnRH-Rezeptors, die in Hypophyse und Hypothalamus durch GnRH aktiviert werden, analysiert. Obwohl Phospholipase C (PLC), Protein Kinase C (PKC) oder Adenylatcyclase in den Tumorzellen pharmakologisch aktiviert werden konnten, hatten GnRH und seine Analoga keinen Einfluß auf PLC, PKC oder Adenylatcyclase. Das deutet darauf hin, daß in den Tumorzellen ein anderer Signaltransduktionsmechanismus vorliegen muß. Anders, als in Hypophyse und Hypothalamus, wird das Signal der GnRH-Rezeptor Aktivierung in den Tumorzellen durch G-Protein αi und nicht durch G-Protein αq weitergeleitet. Ein wichtiger Mechanismus ist die Interaktion des GnRH-Rezeptors mit der durch Wachstumsfaktoren vermittelten mitogenen Signaltransduktion. Mittels eines p42/p44 MAP-Kinase Assays konnte gezeigt werden, daß die durch EGF induzierte MAP-Kinase Aktivität durch GnRH-Analoga komplett unterdrückt werden kann. Quantitative RT-PCR zeigte, daß GnRH-Analoga in der Lage sind, die EGF-induzierte Expression des immediate early response genes *c-fos* dosisabhängig in allen untersuchten Ovarial- und Endometriumkarzinomzellinien mit GnRH-Rezeptor Expression zu inhibieren. Nanomolare Konzentration reichen aus, die *c-fos* Expression auf ein basales Niveau nicht proliferierender Zellen zu drücken. GnRH steuert das Wachstum der Tumorzellen durch Interaktion des GnRH-Rezeptors mit der mitogenen Signaltransduktion der Wachstumsfaktoren.

### Aktivierung des Transkriptionsfaktors NFκB durch GnRH-Analoga in Ovarialund Endometriumkarzinomzellen:

In den Ovarialkarzinomzellinien EFO-21, EFO-27 ***(Gründker et al. 2000)*** und NIH:OVCAR-3 sowie in den Endometriumkarzinomzellinien Hec-1A, Hec-1B und Ishikawa wurde zum ersten Mal beobachtet, daß GnRH-Analoga in der Lage sind, den Transkriptionsfaktor NFκB rezeptor-vermittelt zu aktivieren. Dabei erhöht sich die Aktivität von NFκB um das 5- bis 8-fache. Diese Aktivierung wird über das G-Protein αi vermittelt und ist durch Pertussis Toxin hemmbar. In Kontrollexperimenten mit Zellinien ohne GnRH-Rezeptor Expression wird NFκB durch GNRH nicht aktiviert. Uns stehen zwei verschieden Klone der SK-OV-3 Zellinie zur Verfügung, von denen nur einer den GnRH-Rezeptor exprimiert. Nur in diesem Klon läßt sich NFκB durch GnRH aktivieren.

### Aktivierung des Transkriptionsfaktors NFκB durch GnRH-Analoga in Normalzellen:

Der Nachweis, daß NFκB in den GnRH Rezeptor positiven gonadotropen Hypophysenzellen durch GnRH Analoga nicht aktiviert werden kann wurde mit der immortalisierten Maus Hypophysenzellinie αT3-1 geführt. Da sich die Signaltransduktion des GnRH-Rezeptors in der Hypophyse sich grundsätzlich von derjenigen in den Tumorzellen unterscheidet, gehen wir daher davon aus, daß GnRH Analoga in den Hypophysenzellen keine Wirkung auf NFκB besitzt. In der 3T3 Fibroblastenzellinie ohne GnRH Rezeptor Expression wurde NFκB niemals durch GnRH-Analoga aktiviert. Nur in den GnRH Rezeptor positiven Tumorzellen nicht aber in GnRH Rezeptor positiven wie negativen Normalzellen ist NFκB durch GnRH aktivierbar. Diese Tatsache läßt das Therapiekonzept, über den GnRH Rezeptor NFκB zu aktivieren um dessen Kontrolle ein therapeutisches Gen zu aktivieren, vielversprechend aussehen.

### In vivo Transfektion mit NFκB-LacZ und Aktivierung des Transkriptions-faktors NFκB durch GnRH-Analoga in vivo:

Mit den folgenden Experimenten sollte gezeigt werden, daß die Expression eines eingebrachten Effektorgens unter Kontrolle des NFκB Response Elementes in vivo in GnRH Rezeptor positiven Tumoren durch GnRH Analoga induziert werden kann. Gleichzeitig sollten die Experimente zeigen, daß in Normalgeweben keine NFκB vermittelte Expression des Effektorgens induziert wird. Immundefizienten Nacktmäusen wurden Hec-1B Endometrium-karzinomzellen intraperitoneal verabreicht. Nach Anwachsen der Tumorzellen wurde den Mäusen das Reportergen Plasmid NFKB-LacZ in Kombination mit dem Transfektionsreagenz Lipofect intraperitoneal appliziert. Nach 24 Stunden wurde den Kontrollmäusen Kochsalzlösung und den Versuchsmäusen der GnRH-Agonist Triptorelin intravenös injiziert. Weitere 24 Stunden später wurden die Tiere getötet, die Organe sowie die Tumore entnommen und anschließend mit X-Gal gefärbt. Nur Tumore aus Mäusen mit Triptorelin-Behandlung waren blau gefärbt. Ohne Triptorelin wurde NFκB-LacZ nicht aktiviert. Ovar und Uterus waren sowohl mit als auch ohne Triptorelin blau gefärbt. Alle anderen Organe (Gehirn, Herz, Lunge, Milz, Leber, Niere, Magen, Darm) wurden weder mit noch ohne Triptorelin angefärbt. Dieses Ergebnis zeigt, daß GnRH-Analoga das NFκB nur in den Endometriumkarzinomzellen aktivieren. In Ovar und Uterus scheint NFκB konstitutiv aktiv zu sein. Dies ist aber vernachlässigbar, da diese Organe bei Ovar- und Endometriumkarzinom ohnehin entfernt werden. In alle anderen untersuchten Organen wird NFκB nicht aktiviert, solange Entzündungen dieser Organe vermieden werden. Allein durch liposomale Transfektion NFκB-vermittelt konnte das Reportergen LacZ im Tumor exprimiert werden. Alle anderen Organe mit Ausnahme von Ovar und Uterus zeigten keine Expression. Diese Ergebnisse lassen den Schluß zu, daß das angestrebte Therapiekonzept auch in vivo funktionieren wird.

### In vitro Evaluation des Therapiekonzeptes mit dem therapeutischen Konstrukt NFκB-Tymidinkinase (TK) und dem Wirkstoff (Prodrug) Ganciclovir:

Mit den folgenden Experimenten sollte gezeigt werden, daß die Expression eines eingebrachten therapeutischen Gens unter Kontrolle des NFκB Response Elementes in vitro in GnRH Rezeptor positiven Tumorzellen durch GnRH Analoga induziert werden kann. In den GnRH Rezeptor-positiven Ovarialkarzinomzellinien EFO-21, EFO-27 und NIH:OVCAR-3 sowie in den GnRH Rezeptor-positiven Endometriumkarzinomzellinien Hec-1A, Hec-1B und Ishikawa konnte gezeigt werden, daß in NFκB-TK transfizierten Tumorzellen durch Triptorelin die Thymidinkinase exprimiert und aktiviert wird. Die Thymidinkinase phosphoryliert dann Ganciclovir, welches anschließend von zellulären Enzymen weiter phosphoryliert wird. Erst dadurch wird Ganciclovir toxisch. Dies hatte zur Folge, daß die mit NFκB-TK transfizierten GnRH Rezeptor positiven Tumorzellen nach Gabe von Triptorelin (Aktivierung) und Ganciclovir (Prodrug) starben. Ohne Triptorelin oder ohne Ganciclovir zeigte sich kein Effekt. GnRH Rezeptor-negative Zellen wurden durch diese Therapie nicht geschädigt.

### Literatur:

Gründker C, Schulz K, Günthert AR, Emons G (2000) Luteinizing hormone-releasing hormone induces nuclear factor κB-activation and inhibits apoptosis in ovarian cancer cells. *Journal of Clinical Endocrinology and Metabolism* 85:3815-3820

Gründker C, Günthert AR, Westphalen S, Emons G (2002) Biology of GnRH systems in human gynecological cancers. *European Journal of Endocrinology* 146:1-14

## Patentansprüche

1. Nukleinsäurekonstrukt, welches ein therapeutisches Gen, dessen Genprodukt in einer Zelle die Erzeugung eines zytotoxischen oder therapeutischen Wirkstoffs auslöst, unter Kontrolle eines NFκB-spezifischen Promotors enthält.

2. Nukleinsäurekonstrukt nach Anspruch 1, **dadurch gekennzeichnet, dass** der NFκBspezifische Promotor wenigstens eine Kopie des kappaB-Enhancers, vorzugsweise 4 bis 6 Kopien des KappaB-Enhancers, fusioniert an einen für die Aktivierung des therapeutischen Gens geeigneten Promotors enthält.

3. Nukleinsäurekonstrukt nach Anspruch 1 oder 2, **dadurch gekennzeichnet, dass** das therapeutische Gen das Herpes simplex Virus (HSV)-Thymidinkinase-Gen ist.

4. Nukleinsäurekonstrukt nach Anspruch 1 oder 2, **dadurch gekennzeichnet, dass** das therapeutische Gen das Varicella zoster Virus-Thymidinkinase-Gen ist.

5. Nukleinsäurekonstrukt nach Anspruch 1 oder 2, **dadurch gekennzeichnet, dass** das therapeutische Gen das Echerichia coli-Cytosindeaminase-Gen ist.

6. Nukleinsäurekonstrukt nach einem der Ansprüche 1 bis 5, **dadurch gekennzeichnet, dass** es stromabwärts des therapeutischen Gens wenigstens eine polyA-Sequenz enthält

7. Nukleinsäurekonstrukt nach einem der Ansprüche 1 bis 6, **dadurch gekennzeichnet, dass** es zusätzlich eine Sequenz für die Expression des Wirkstoff-Vorläufers in der Zielzelle enthält.

8. Nukleinsäurekonstrukt nach einem der Ansprüche 1 bis 7, **dadurch gekennzeichnet, dass** das Nukleinsäurekonstrukt ein Plasmid ist.

9. Vektor für die Transfektion von Zielzellen mit dem Nukleinsäurekonstrukt, **dadurch gekennzeichnet, dass** der Vektor ein liposomaler Vektor ist, der das Nukleinsäurekonstrukt nach einem der Ansprüche 1 bis 8 enthält.

10. Vektor für die Transfektion von Zielzellen mit dem Nukleinsäurekonstrukt, **dadurch gekennzeichnet, dass** der Vektor ein viraler Vektor ist, vorzugsweise ein retroviraler oder adenoviraler Vektor, der das Nukleinsäurekonstrukt nach einem der Ansprüche 1 bis 8 enthält.

11. Verwendung des Nukleinsäurekonstrukts oder des Vektors nach einem der Ansprüche 1 bis 10 für die Herstellung eines Präparats zur Behandlung von GnRH-Rezeptor-positiven Tumoren.

12. Verwendung nach Anspruch 11 oder 12, **dadurch gekennzeichnet, dass** der GnRH-positive Tumor ein Endometrium-, Ovarial, Mamma- oder Prostatakarzinom ist.

13. Verwendung nach Anspruch 12, **dadurch gekennzeichnet dass** das Präparat das Nukleinsäurekonstrukt oder den Vektor mit dem Nukleinsäurekonstrukt in einer Injektionslösung oder einer Infusionslösung enthält.

14. Verwendung nach Anspruch 12 oder 13, **dadurch gekennzeichnet, dass** das Präparat mit dem Nukleinsäurekonstrukt oder dem Vektor zusätzlich einen Wirkstoff-Vorläufer enthält oder für die Co-Applikation mit dem Wirkstoff-Vorläufer vorgesehen ist.

15. Verwendung nach einem der Ansprüche 12 bis 14, **dadurch gekennzeichnet, dass** das Präparat mit dem Nukleinsäurekonstrukt oder dem Vektor für die zeitlich aufeinander abgestimmte Applikation in Verbindung mit GnRH oder einem GnRH-Analogon und gegebenenfalls dem Wirkstoff-Vorläufer vorgesehen ist.
